# EUROPEAN PATENT SPECIFICATION

(11) **EP 4 279 876 B1**
(45) Date of publication and mention of the grant of the patent: **18.12.2024**
(21) Application number: 23172189.5
(22) Date of filing: 09.05.2023
(51) Int. Cl.: G01D 9/00, G05B 23/02, G05B 19/042

(54) **DATA RECORDING APPARATUS, DATA RECORDING METHOD, AND DATA RECORDING PROGRAM**
DATENAUFZEICHNUNGSVORRICHTUNG, DATENAUFZEICHNUNGSVERFAHREN UND DATENAUFZEICHNUNGSPROGRAMM
APPAREIL D'ENREGISTREMENT DE DONNÉES, PROCÉDÉ D'ENREGISTREMENT DE DONNÉES ET PROGRAMME D'ENREGISTREMENT DE DONNÉES

(30) Priority: 17.05.2022 JP 2022080799
(43) Date of publication of application: 22.11.2023
(73) Proprietor: Yokogawa Electric Corporation, Musashino-shi, Tokyo 180-8750 (JP)
(72) Inventor: YOSHIDA, Yoshitaka, Tokyo, 180-8750 (JP); EMA, Nobuaki, Tokyo, 180-8750 (JP)
(74) Representative: Stöckeler, Ferdinand

(56) References cited:
- EP-A1- 3 879 470
- EP-A1- 3 885 927
- EP-A1- 3 926 430
- EP-A1- 3 933 532
- EP-A1- 3 951 535
- JP-A- 2021 196 316

## Description

### BACKGROUND

### 1. TECHNICAL FIELD

The present invention relates to a data recording apparatus, a data recording method, and a data recording program.

### 2. RELATED ART

JP 2021-196 316 A discloses that "a part of measurement data from a target sensor is deleted on the basis of measurement data from another sensor to reduce amount of data to be recorded". EP 3 933 532 A1, EP 3 951 535 A1, EP 3 879 470 A1, and EP 3 885 927 A1 each discloses a data management system.

### SUMMARY

According to a first aspect of the present invention, a data recording apparatus according to claim 1 is provided.
The data recording apparatus includes: a data acquisition unit configured to acquire measurement data obtained by measuring a measurement target from a plurality of sensors; a data prediction unit configured to predict measurement data acquired from at least any one sensor of the plurality of sensors by using measurement data acquired from another sensor among the plurality of sensors; a decision unit configured to decide a set of sensors capable of reproducing the measurement data acquired from the plurality of sensors within a predetermined allowable range by using the measurement data acquired by the data acquisition unit and the measurement data predicted by the data prediction unit, wherein measurement data acquired from a sensor not included in the set can be reproduced within the predetermined allowable range by using the measurement data acquired from one or more sensors in the set; a data selection unit configured to select, from among the measurement data acquired from the plurality of sensors, measurement data acquired from a sensor included in the set in preference to measurement data acquired from a sensor not included in the set; and a data recording unit configured to record the measurement data selected by the data selection unit, wherein the measurement data selected by the data selection unit include the measurement data acquired from sensors included in the set.

In the data recording apparatus, the decision unit may have an assignment unit configured to assign, to the another sensor, identification information indicating whether a prediction value, which is obtained by predicting the measurement data acquired from the at least any one sensor by using the measurement data acquired from the another sensor, is within the allowable range, and a sensor which is assigned with the identification information indicating that the prediction value is within the allowable range may be included in the set.

In any one of the data recording apparatuses, the decision unit may have: a generation unit configured to generate a plurality of candidate sets of sensors capable of reproducing the measurement data acquired from the plurality of sensors within a predetermined allowable range; and a set selection unit configured to select the set from the plurality of candidate sets according to a predetermined criterion.

In any one of the data recording apparatuses,, the generation unit may be configured to generate the plurality of candidate sets on a basis of a round robin of patterns in which a prediction value obtained by predicting the measurement data acquired from the at least any one sensor using the measurement data acquired from the another sensor is within the allowable range.

In any one of the data recording apparatuses, the set selection unit may be configured to select, among the plurality of candidate sets, a candidate set in which an amount of the measurement data to be recorded is small in preference to a candidate set in which the amount is large.

In any one of the data recording apparatuses, the set selection unit may be configured to select, among the plurality of candidate sets, a candidate set in which a difference between the measurement data acquired by the data acquisition unit and the measurement data predicted by the data prediction unit is small in preference to a candidate set in which the difference is large.

In any one of the data recording apparatuses, the allowable range may be settable to a range different for each sensor.

In any one of the data recording apparatuses, the data recording unit may be configured to delete the measurement data acquired from the sensor not included in the set and record only the measurement data acquired from the sensor included in the set.

In any one of the data recording apparatuses, the data recording unit may be configured to record measurement data acquired from the sensor not included in the set after reducing the number of pieces of data per unit time.

In any one of the data recording apparatuses, the data recording unit may be configured to record measurement data acquired from the sensor not included in the set after reducing a data size per piece of data.

In any one of the data recording apparatuses, the data recording unit may be configured to record the measurement data selected by the data selection unit in response to a remaining capacity available for recording falling below a predetermined threshold value.

In any one of the data recording apparatuses, the data recording unit may be configured to record the measurement data selected by the data selection unit in response to elapse of predetermined time.

Any one of the data recording apparatuses may further include a data transmission unit configured to transmit the measurement data selected by the data selection unit to another system or apparatus.

According to a second aspect of the present invention, a data recording method according to claim 14 is provided.
The data recording method that, when executed by a computer, causes the computer to perform operations includes: acquiring measurement data obtained by measuring a measurement target from a plurality of sensors; predicting measurement data acquired from at least any one sensor of the plurality of sensors by using measurement data acquired from another sensor among the plurality of sensors; deciding a set of sensors capable of reproducing the measurement data acquired from the plurality of sensors within a predetermined allowable range by using the measurement data acquired by the acquiring and the measurement data predicted by the predicting, wherein measurement data acquired from a sensor not included in the set can be reproduced within the predetermined allowable range by using the measurement data acquired from one or more sensors in the set; selecting, from among the measurement data acquired from the plurality of sensors, measurement data acquired from a sensor included in the set in preference to measurement data acquired from a sensor not included in the set; and recording the measurement data selected by the selecting, wherein the measurement data selected by the selecting include the measurement data acquired from sensors included in the set.

According to a third aspect of the present invention, a data recording program according to claim 15 is provided.
The data recording program that, when executed by a computer, may cause the computer to function as: a data acquisition unit configured to acquire measurement data obtained by measuring a measurement target from a plurality of sensors; a data prediction unit configured to predict measurement data acquired from at least any one sensor of the plurality of sensors by using measurement data acquired from another sensor among the plurality of sensors; a decision unit configured to decide a set of sensors capable of reproducing the measurement data acquired from the plurality of sensors within a predetermined allowable range by using the measurement data acquired by the data acquisition unit and the measurement data predicted by the data prediction unit, wherein measurement data acquired from a sensor not included in the set can be reproduced within the predetermined allowable range by using the measurement data acquired from one or more sensors in the set; a data selection unit configured to select, from among the measurement data acquired from the plurality of sensors, measurement data acquired from a sensor included in the set in preference to measurement data acquired from a sensor not included in the set; and a data recording unit configured to record the measurement data selected by the data selection unit, wherein the measurement data selected by the data selection unit (150) include the measurement data acquired from sensors included in the set.

The summary does not necessarily describe all necessary features of the embodiments of the present invention. In addition, the present invention may also be a subcombination of the features described above but the invention is solely defined by the appended claims.

### BRIEF DESCRIPTION OF THE DRAWINGS

Fig. 1 illustrates an example of a block diagram of a data recording apparatus 100 according to the present embodiment.
Fig. 2 illustrates an example of a flow of a data recording method by the data recording apparatus 100 according to the present embodiment.
Fig. 3 illustrates an example of a flow in which a decision unit 140 of the data recording apparatus 100 according to the present embodiment decides a set of sensors.
Fig. 4 illustrates an example of a block diagram of the data recording apparatus 100 according to a modification of the present embodiment.
Fig. 5 illustrates an example of a round robin of patterns which may be considered by the data recording apparatus 100 according to the modification of the present embodiment.
Fig. 6 illustrates an example of a block diagram of the data recording apparatus 100 according to another modification of the present embodiment.
Fig. 7 illustrates an example of a computer 9900 in which a plurality of aspects of the present invention may be embodied in whole or in part.

### DESCRIPTION OF EXEMPLARY EMBODIMENTS

Hereinafter, the present invention will be described through embodiments of the invention, but the following embodiments do not limit the claimed invention. In addition, some combinations of features described in the embodiments may not be essential for the solving means of the invention.

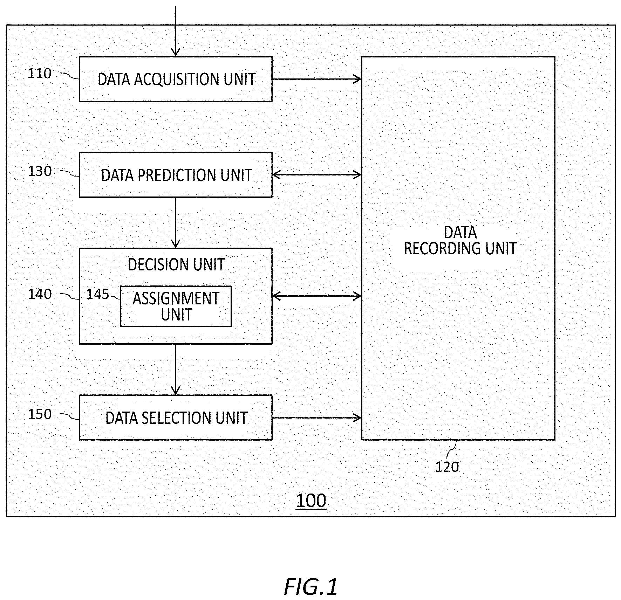

Fig. 1 illustrates an example of a block diagram of a data recording apparatus 100 according to the present embodiment. The data recording apparatus 100 acquires measurement data obtained by measuring a measurement target from a plurality of sensors and records the measurement data. Then, the data recording apparatus 100 decides a set of sensors capable of reproducing the acquired measurement data within an allowable range, and selectively records the acquired measurement data according to the set.

The data recording apparatus 100 may be a computer such as a personal computer (PC), a tablet computer, a smartphone, a workstation, a server computer, or a general-purpose computer, or may be a computer system in which a plurality of computers are connected. Such a computer system is also a computer in a broad sense. In addition, the data recording apparatus 100 may also be implemented by one or more virtual computer environments executable in a computer. Alternatively, the data recording apparatus 100 may be a dedicated computer designed for recording data or may be dedicated hardware realized by a dedicated circuit. In addition, when connection to the Internet is possible, the data recording apparatus 100 may be realized by cloud computing.

The data recording apparatus 100 includes a data acquisition unit 110, a data recording unit 120, a data prediction unit 130, a decision unit 140, and a data selection unit 150. Note that, these blocks are functional blocks that are each functionally divided, and may not be necessarily required to be matched with actual apparatus configurations. That is, in the present drawing, an apparatus indicated by one block may not be necessarily required to be configured by one apparatus. In addition, in the present drawing, apparatuses indicated by separate blocks may not be necessarily required to be configured by separate apparatuses. The same applies to subsequent block diagrams.

The data acquisition unit 110 acquires measurement data obtained by measuring a measurement target from a plurality of sensors. The data acquisition unit 110 supplies the acquired measurement data to the data recording unit 120.

The data recording unit 120 records the measurement data acquired by the data acquisition unit 110. In addition, the data recording unit 120 records the measurement data selected by the data selection unit 150 to be described later.

The data prediction unit 130 predicts the measurement data acquired from at least any one sensor of the plurality of sensors by using the measurement data acquired from another sensor among the plurality of sensors. The data prediction unit 130 supplies the predicted measurement data (a prediction value obtained by predicting the measurement data) to the decision unit 140.

The decision unit 140 uses the measurement data acquired by the data acquisition unit 110 and the measurement data predicted by the data prediction unit 130 to decide a set of sensors capable of reproducing the measurement data acquired from the plurality of sensors within a predetermined allowable range.

In the present embodiment, the decision unit 140 includes an assignment unit 145. The assignment unit 145 assigns, to another sensor, identification information indicating whether the prediction value, which is obtained by predicting the measurement data acquired from at least any one sensor by using the measurement data acquired from another sensor, is within the allowable range. Then, the decision unit 140 decides a set of sensors according to the identification information. This will be described below in detail. The decision unit 140 supplies, to the data selection unit 150, information indicating the decided set of sensors.

The data selection unit 150 selects, from among the measurement data acquired from the plurality of sensors, the measurement data acquired from the sensor included in the set decided by the decision unit 140 in preference to the measurement data acquired from the sensor not included in the set. The data selection unit 150 supplies, to the data recording unit 120, information indicating the selected result. In response to this, the data recording unit 120 records the measurement data selected by the data selection unit 150.

A method in which the data recording apparatus 100 including such a functional unit records the measurement data will be described in detail by using a flow.

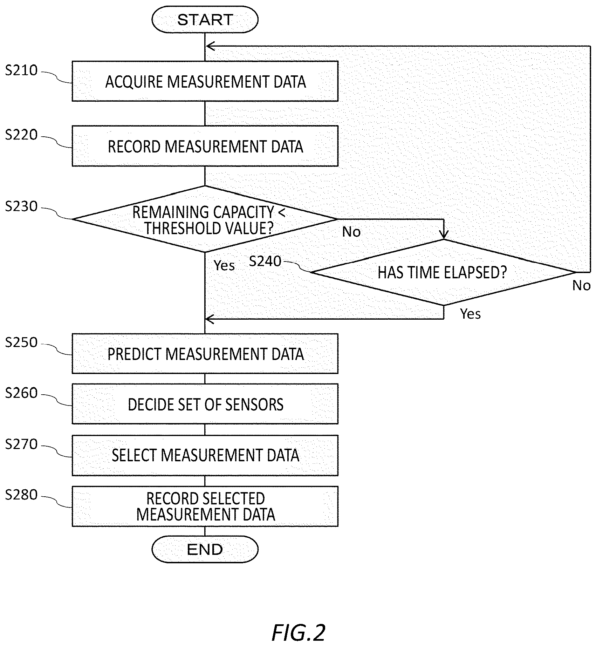

Fig. 2 illustrates an example of a flow of a data recording method by the data recording apparatus 100 according to the present embodiment.

In step S210, the data recording apparatus 100 acquires measurement data. For example, the data acquisition unit 110 acquires measurement data obtained by measuring a measurement target from each of a plurality of sensors in time series via a communication network.

Such communication network may be a network connecting a plurality of computers. For example, the communication network may be a global network interconnecting a plurality of computer networks, and, as an example, the communication network may be the Internet using the Internet Protocol or the like. Instead, the communication network may be realized by a dedicated line. Specifically, the data acquisition unit 110 may directly or indirectly conduct transactions with a mobile phone, a smartphone, a fourth-generation (4G) terminal, a fifth-generation (5G) terminal, or the like, to acquire the measurement data.

Note that, in the above description, a case where the data acquisition unit 110 acquires the measurement data from the plurality of sensors via the communication network has been described as an example, but the present invention is not limited thereto. For example, the data acquisition unit 110 may acquire the measurement data from the plurality of sensors via another means, such as a user input or various memory devices, different from the communication network.

Herein, the plurality of sensors can acquire measurement data obtained by measuring a measurement target. Such a plurality of sensors may be, for example, a sensor (for example, a process control (measurement) sensor) or an Internet of Things (IoT) sensor installed in an operational technology (OT) region, and may be, as an example, an industrial sensor connected to or integrally constituted with one or more field instruments provided in a plant.

Herein, such plant may be, for example, besides an industrial plant such as a chemical plant, a plant for managing and controlling a wellhead or its surrounding area of a gas field, an oil field or the like, a plant for managing and controlling power generation such as hydraulic power generation, thermal power generation and nuclear power generation, a plant for managing and controlling energy harvesting such as solar photovoltaic generation, wind power generation or the like, and a plant for managing and controlling water and sewerage, a dam or the like.

In addition, a field instrument provided in such plant may be, for example, a pressure gauge, a flow meter, a sensor instrument such as a temperature sensor, a valve instrument such as a flow control valve or an on-off valve, an actuator instrument such as a fan or a motor, an imaging instrument such as a camera or a video camera for imaging a situation or an object in the plant, an audio instrument such as a microphone or a speaker for collecting noises or the like in the plant or emitting an alarm or the like, a position detection instrument for outputting position information of each instrument, or the like.

Therefore, the data acquisition unit 110 may acquire measurement data, such as temperature, pressure, flow rate, acceleration, magnetic field, position, camera image, switch on/off data, sound, and a combination thereof, measured by the sensor itself, or measurement data measured inside the field instrument. In addition, the data acquisition unit 110 may acquire, as the measurement data, a value generated by using a formula based on these data. The data acquisition unit 110 supplies the acquired measurement data to the data recording unit 120.

In step S220, the data recording apparatus 100 records the measurement data. For example, the data recording unit 120 records the measurement data acquired from the plurality of sensors in step S210 in time series for each sensor in the order of measurement by each sensor.

In step S230, the data recording apparatus 100 determines whether a remaining capacity available for recording measurement data falls below a predetermined threshold value. For example, the data recording apparatus 100 calculates the remaining capacity available for recording measurement data by subtracting the total capacity of the measurement data recorded in step S220 from the full capacity available for recording measurement data. Then, the data recording apparatus 100 compares the calculated remaining capacity with the threshold value. As a result of the comparison, if it is determined that the remaining capacity does not fall below the threshold value (No), the data recording apparatus 100 advances the processing to step S240.

In step S240, the data recording apparatus 100 determines whether predetermined time has elapsed. At this time, the data recording apparatus 100 may use, as a starting point of the elapse of time, a time point at which the measurement data is first recorded in step S220 or a time point at which the measurement data recorded in step S220 is last accessed, for example. The data recording apparatus 100 compares the elapsed time with the predetermined time. As a result of the comparison, if it is determined that the predetermined time has not elapsed (No), the data recording apparatus 100 returns the processing to step S210 and continues the flow.

On the other hand, if it is determined in step S230 that the remaining capacity falls below the threshold value (Yes), or if it is determined in step S240 that the predetermined time has elapsed (Yes), the data recording apparatus 100 advances the processing to step S250.

In step S250, the data recording apparatus 100 predicts the measurement data. For example, the data prediction unit 130 accesses the data recording unit 120 and acquires the measurement data recorded in step S220. Then, the data prediction unit 130 predicts the measurement data acquired from at least any one sensor of the plurality of sensors by using the measurement data acquired from another sensor among the plurality of sensors.

Herein, a value of the measurement data acquired from the sensor is referred to as an "actual measurement value", and a value obtained by predicting the measurement data is referred to as a "prediction value". In this case, for example, when predicting the measurement data acquired from the sensor to be predicted (that is, "at least any one sensor"), the data prediction unit 130 may substitute the actual measurement value of the sensor as a prediction source (that is, "another sensor") into a predetermined formula, thereby calculating the prediction value of the sensor to be predicted.

As an example, it is assumed that the data recording apparatus 100 has acquired the measurement data of the sensors 1 to 5. Then, it is assumed that it is known that the actual measurement value of the sensor 1 correlates with the actual measurement value of the sensor 2 and the actual measurement value of the sensor 3. In this case, when predicting the measurement data acquired from the sensor 1 to be predicted, the data prediction unit 130 may substitute the actual measurement value of the sensor 2 and the actual measurement value of the sensor 3, which are prediction sources, into the predetermined formula, thereby calculating the prediction value of the sensor 1. Similarly, as an example, it is assumed that it is known that the actual measurement value of the sensor 4 correlates with the actual measurement value of the sensor 5. In this case, when predicting the measurement data acquired from the sensor 4 to be predicted, the data prediction unit 130 may substitute the actual measurement value of the sensor 5 as the prediction source into the predetermined formula, thereby calculating the prediction value of the sensor 4.

At this time, the sensor to be predicted and the sensor as the prediction source do not need to be the same in the type of the physical quantity to be measured, and may be different from each other. For example, the data prediction unit 130 can also calculate the prediction value of a pressure P by substituting the actual measurement value of a flow rate F and the actual measurement value of a temperature T into the predetermined formula.

Note that, in the above description, a case where the correlation between the measurement data acquired from the plurality of sensors is known has been described as an example, but the present invention is not limited thereto. The correlation between the measurement data acquired from the plurality of sensors may vary depending on, for example, the installation environment of the sensor. In such a case, the data prediction unit 130 may perform, for example, regression analysis on the measurement data acquired from the plurality of sensors. As an example, the data prediction unit 130 may calculate a regression coefficient such that a difference between the prediction value of the sensor to be predicted and the value obtained by multiplying the actual measurement value of the sensor as the prediction source by the regression coefficient is minimized. Then, by using the regression coefficient having the minimum difference, the data prediction unit 130 may calculate the prediction value of the sensor to be predicted. The data prediction unit 130 supplies the predicted measurement data (prediction value) to the decision unit 140.

In step S260, the data recording apparatus 100 decides a set of sensors. For example, the decision unit 140 accesses the data recording unit 120 and acquires the measurement data recorded in step S220. Then, the decision unit 140 uses the acquired measurement data and the measurement data predicted in step S250 to decide a set of sensors capable of reproducing the measurement data acquired from the plurality of sensors within a predetermined allowable range. At this time, in the present embodiment, the assignment unit 145 assigns, to another sensor, identification information indicating whether a prediction value, which is obtained by predicting measurement data acquired from at least any one sensor by using the measurement data acquired from another sensor, is within the allowable range. Then, the decision unit 140 decides a set of sensors by including a sensor, which is assigned with the identification information indicating that the prediction value is within the allowable range, in the set. This will be described below in detail. The decision unit 140 supplies information indicating the decided set of sensors to the data selection unit 150.

In step S270, the data recording apparatus 100 selects measurement data. For example, the data selection unit 150 selects, from among the measurement data acquired from the plurality of sensors, the measurement data acquired from the sensor included in the set decided in step S260 in preference to the measurement data acquired from the sensor not included in the set. As an example, when the sensor 2, the sensor 3, and the sensor 5 are included in the decided set, the data selection unit 150 selects the measurement data acquired from the sensor 2, the sensor 3, and the sensor 5 included in the set in preference to the measurement data acquired from the sensor 1 and the sensor 4 not included in the set.

At this time, the data selection unit 150 may select only the measurement data acquired from the sensor 2, the sensor 3, and the sensor 5 included in the set, and may not select the measurement data acquired from the sensor 1 and the sensor 4 not included in the set. Alternatively, the data selection unit 150 may select, as data with a priority of "high", the measurement data acquired from the sensor 2, the sensor 3, and the sensor 5 included in the set, and select, as data with a priority of "low", the measurement data acquired from the sensor 1 and the sensor 4 not included in the set. The data selection unit 150 supplies, to the data recording unit 120, information indicating the selected result.

In step S280, the data recording apparatus 100 records the selected measurement data. For example, the data recording unit 120 records the measurement data selected in step S270. As an example, when only the measurement data acquired from the sensor 2, the sensor 3, and the sensor 5 is selected in step S270, the data recording unit 120 may delete the measurement data acquired from the sensor 1 and the sensor 4 and record only the measurement data acquired from the sensor 2, the sensor 3, and the sensor 5. For example, in this manner, the data recording unit 120 can also delete the measurement data acquired from the sensors not included in the set and record only the measurement data acquired from the sensors included in the set.

In addition, when the measurement data acquired from the sensor 2, the sensor 3, and the sensor 5 is selected as the data with the priority of "high" and the measurement data acquired from the sensor 1 and the sensor 4 is selected as the data with the priority of "low" in step S270, the data recording unit 120 may reduce the number of pieces of data per unit time (thinning out the number of samples in a time axis direction) and record the measurement data acquired from the sensor 1 and the sensor 4 with the low priority. For example, in this manner, the data recording unit 120 can also record the measurement data acquired from the sensor not included in the set after reducing the number of pieces of data per unit time.

In addition, when the measurement data acquired from the sensor 2, the sensor 3, and the sensor 5 is selected as the data with the priority of "high" and the measurement data acquired from the sensor 1 and the sensor 4 is selected as the data with the priority of "low" in step S270, the data recording unit 120 may record the measurement data acquired from the sensor 1 and the sensor 4 with the low priority after reducing the data size per piece of data (reducing the bit width (quantization bit depth) in the magnitude axis direction). For example, in this manner, the data recording unit 120 can also record the measurement data acquired from the sensor not included in the set after reducing the data size per piece of data.

For example, in this manner, the data recording apparatus 100 selectively records the measurement data and ends the flow. In this manner, the data recording unit 120 may record the selected measurement data in response to the remaining capacity available for recording falling below the predetermined threshold value (in response to the determination of Yes in step S230). In addition, the data recording unit 120 may record the selected measurement data in response to the elapse of the predetermined time (in response to the determination of Yes in step S240).

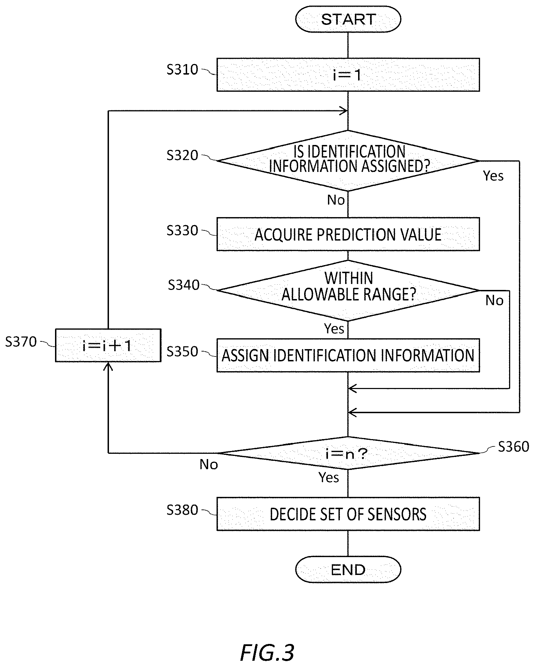

Fig. 3 illustrates an example of a flow in which the decision unit 140 of the data recording apparatus 100 according to the present embodiment decides a set of sensors. In this flow, i represents an index of the sensor. In addition, n represents the number of sensors, and for example, when the data recording apparatus 100 acquires the measurement data of the sensors 1 to 5, n is 5.

In step S310, the decision unit 140 sets i to 1, which is an initial value. In the case of i = 1, the sensor 1 is a target sensor.

In step S320, the decision unit 140 determines whether the identification information indicating that the prediction value is within the allowable range is assigned to the target sensor. In the case of i = 1, the decision unit 140 determines whether the identification information indicating that the prediction value is within the allowable range is assigned to the sensor 1. At this time point, since such identification information is not assigned to any sensor, the determination result is "No". In this case, the decision unit 140 advances the processing to step S330.

In step S330, the decision unit 140 acquires the prediction value of the target sensor. In the case of i = 1, the decision unit 140 acquires, from the data prediction unit 130, a prediction value obtained by predicting the measurement data acquired from the sensor 1 which is the target sensor by using measurement data acquired from at least any one of the sensors 2 to 5 which are other sensors. As an example, it is assumed that the data prediction unit 130 calculates a prediction value obtained by predicting the actual measurement value of the measurement data acquired from the sensor 1 by using the actual measurement values of the measurement data acquired from the sensor 2 and the sensor 3. In this case, the decision unit 140 may acquire the prediction value as the prediction value of the sensor 1. Note that, when the data prediction unit 130 has calculated a plurality of prediction values by using the measurement data acquired from (a combination of) different sensors, the decision unit 140 may acquire a plurality of calculated prediction values.

In step S340, the decision unit 140 determines whether the prediction value acquired in step S330 is within the allowable range. Herein, the allowable range may be defined by, for example, an error of the prediction value with respect to the actual measurement value, that is, a difference between the actual measurement value and the prediction value. As an example, the allowable range may be defined to be within an error of 1%, within an error of 5%, or the like. Such an allowable range can be set to a range different for each sensor. That is, for example, the allowable range may be set within an error of 1% for the sensor 1, and the allowable range may be set within the error of 5% for the sensor 4.

If it is determined that the prediction value (when there are a plurality of prediction values, any of the plurality of prediction values) is not within the allowable range (No), the decision unit 140 advances the processing to step S360. That is, the decision unit 140 omits the processing of step S350. On the other hand, if it is determined that the prediction value (when there is a plurality of prediction values, any of the plurality of prediction values) is within the allowable range (Yes), the decision unit 140 advances the processing to step S350. In the case of i = 1, for example, when the prediction value obtained by predicting the measurement data acquired from the sensor 1 by using the measurement data acquired from the sensors 2 and 3 is within an error of 1%, the determination result is "Yes". In this case, the decision unit 140 advances the processing to step S350.

In step S350, the assignment unit 145 assigns, to another sensor, the identification information indicating whether the prediction value, which is obtained by predicting the measurement data acquired from at least any one sensor by using the measurement data acquired from another sensor, is within the allowable range. At this time, the assignment unit 145 may use, as the identification information, information capable of identifying, with a binary value, whether the prediction value is within the allowable range, or may use information capable of identifying, with a ternary value or more, within what percentage the error is. Prior to this, a case where the assignment unit 145 uses, as the identification information, a flag indicating that the prediction value is within the allowable range will be described as an example. In the case of i = 1, for example, when the prediction value obtained by predicting the measurement data acquired from the sensor 1 by using the measurement data acquired from the sensors 2 and 3 is within an error of 1%, the assignment unit 145 assigns the flag to the sensors 2 and 3. Then, the decision unit 140 advances the processing to step S360.

In step S360, the decision unit 140 determines whether i is n. That is, the decision unit 140 determines whether the index of the sensor has reached the number of sensors. Thus, the decision unit 140 determines whether all the sensors have become the target sensors. In the case of i = 1, the determination result is "No". In this case, the decision unit 140 advances the processing to step S370.

In step S370, the decision unit 140 increments i such that i = i + 1. In the case of i = 1, the decision unit 140 set i = 2 to set the sensor 2 as the target sensor. Then, the decision unit 140 returns the processing to step S320 and continues the flow.

In step S320, in the case of i = 2, the decision unit 140 determines whether the identification information indicating that the prediction value is within the allowable range is assigned to the sensor 2. Herein, in step S350 described above, since the flag indicating that the prediction value is within the allowable range is assigned to the sensor 2, the determination result is "Yes". In this case, the decision unit 140 advances the processing to step S360. That is, the decision unit 140 omits the processing of step S330 to step S350.

In step S360, in the case of i = 2, the determination result is "No". In this case, the decision unit 140 advances the processing to step S370.

In step S370, in the case of i = 2, the decision unit 140 set i = 3 to set the sensor 3 as the target sensor. Then, the decision unit 140 returns the processing to step S320 and continues the flow.

In step S320, in the case of i = 3, the decision unit 140 determines whether the identification information indicating that the prediction value is within the allowable range is assigned to the sensor 3. Herein, in step S350 described above, since the flag indicating that the prediction value is within the allowable range is assigned to the sensor 3, the determination result is "Yes". In this case, the decision unit 140 advances the processing to step S360.

In step S360, in the case of i = 3, the determination result is "No". In this case, the decision unit 140 advances the processing to step S370.

In step S370, in the case of i = 3, the decision unit 140 set i = 4 to set the sensor 4 as the target sensor. Then, the decision unit 140 returns the processing to step S320 and continues the flow.

In step S320, in the case of i = 4, the decision unit 140 determines whether the identification information indicating that the prediction value is within the allowable range is assigned to the sensor 4. Herein, since the flag indicating that the prediction value is within the allowable range is not assigned to the sensor 4, the determination result is "No". In this case, the decision unit 140 advances the processing to step S330.

In step S330, in the case of i = 4, the decision unit 140 acquires, from the data prediction unit 130, a prediction value obtained by predicting the measurement data acquired from the sensor 4 which is the target sensor by using the measurement data acquired from at least any one of the sensors 1 to 3 and 5 which are other sensors. As an example, it is assumed that the data prediction unit 130 calculates a prediction value obtained by predicting the actual measurement value of the measurement data acquired from the sensor 4 by using the actual measurement value of the measurement data acquired from the sensor 5. In this case, the decision unit 140 may acquire the prediction value as the prediction value of the sensor 4 which is the target sensor.

In step S340, In the case of i = 4, the allowable range (within an error of 5%) set for the sensor 4 which is the target sensor is used. For example, when the prediction value obtained by predicting the measurement data acquired from the sensor 4 by using the measurement data acquired from the sensor 5 is within the error of 5%, the determination result is "Yes". In this case, the decision unit 140 advances the processing to step S350.

In step S350, in the case of i = 4, when the prediction value obtained by predicting the measurement data acquired from the sensor 4 by using the measurement data acquired from the sensor 5 is within the error of 5%, the assignment unit 145 assigns the flag indicating that the prediction value is within the allowable range to the sensor 5. Then, the decision unit 140 advances the processing to step S360.

In step S360, in the case of i = 4, the determination result is No. In this case, the decision unit 140 advances the processing to step S370.

In step S370, in the case of i = 4, the decision unit 140 sets i = 5 to set the sensor 5 as the target sensor. Then, the decision unit 140 returns the processing to step S320 and continues the flow.

In step S320, in the case of i = 5, the decision unit 140 determines whether the identification information indicating that the prediction value is within the allowable range is assigned to the sensor 5. Herein, in step S350 described above, since the flag indicating that the prediction value is within the allowable range is assigned to the sensor 5, the determination result is "Yes". In this case, the decision unit 140 advances the processing to step S360.

In step S360, in the case of i = 5, the determination result is "Yes". In this case, the decision unit 140 advances the processing to step S380.

In step S380, the decision unit 140 decides a set of sensors according to the identification information. For example, the decision unit 140 includes, in the set, a sensor which is assigned with the flag indicating that the prediction value is within the allowable range. As an example, when the flag is assigned to the sensors 2, 3, and 5, the decision unit 140 decides the sensors 2, 3, and 5 as a set of sensors.

Herein, since the measurement data acquired from the sensor 1 can be reproduced within an allowable range (within an error of 1%) by using the measurement data acquired from the sensors 2 and 3, the measurement data can be regarded as data that may be deleted (data having a low priority of recording). On the other hand, since the measurement data acquired from the sensors 2 and 3 is essential for reproducing the measurement data acquired from the sensor 1 within the allowable range, the measurement data can be regarded as data that should not be deleted (data having a high priority of recording). Similarly, since the measurement data acquired from the sensor 4 can be reproduced within an allowable range (within an error of 5%) by using the measurement data acquired from the sensor 5, the measurement data can be regarded as data that may be deleted. On the other hand, since the measurement data acquired from the sensor 5 is essential for reproducing the measurement data acquired from the sensor 4, the measurement data can be regarded as data that should not be deleted.

In other words, even when the measurement data acquired from the sensor 1 and the sensor 4 is deleted from the record, it can be said that the measurement data acquired from the sensors 1 to 5 can be reproduced within the allowable range as long as the measurement data acquired from the sensors 2, 3, and 5 are recorded. Therefore, such a set of sensors including the sensors 2, 3, and 5 can be referred to as a set capable of reproducing the measurement data acquired from the sensors 1 to 5 within a predetermined allowable range. The decision unit 140 decides the set of sensors in this manner, for example, and ends the flow.

Then, the decision unit 140 supplies, to the data selection unit 150, information indicating the set of sensors decided in this manner. In response to this, the data selection unit 150 selects, from among the measurement data acquired from the plurality of sensors, the measurement data acquired from the sensor included in the set in preference to the measurement data acquired from the sensor not included in the set. Then, the data selection unit 150 supplies, to the data recording unit 120, information indicating the selected result. In response to this, the data recording unit 120 records the measurement data selected by the data selection unit 150. Therefore, the data recording apparatus 100 can record the measurement data acquired from the sensor included in the set decided in this manner in preference to the measurement data acquired from the sensor not included in the set.

It is expected that an amount of data will explosively increase, for example, when a process control system in the OT (Operational Technology) region is coupled to a system in the IT (Information Technology) region or the like. In such a situation, it is not practical to record all the data as it is, and it is necessary to reduce the amount of data or sort out the data. Conventionally, it has been studied to select a target sensor available for predicting measurement data by using measurement data acquired from another sensor and to delete the measurement data acquired from the target sensor. In this case, it is possible to delete the measurement data of the target sensor available for prediction from the measurement data of another sensor. However, when the measurement data of another sensor used for prediction (serving as a prediction source) is deleted, a situation occurs in which the measurement data of the target sensor that could have been previously predicted cannot be predicted (reproduced).

On the other hand, the data recording apparatus 100 according to the present embodiment decides a set of sensors capable of reproducing the acquired measurement data, and selectively records the acquired measurement data according to the set. Thus, according to the data recording apparatus 100 according to the present embodiment, it is possible to selectively record the measurement data such that the measurement data acquired from the plurality of sensors can be reproduced without causing a situation in which the measurement data of the target sensor that could have been previously predicted cannot be reproduced.

In addition, the data recording apparatus 100 according to the present embodiment may include, in the set of sensors, a sensor which is assigned with the identification information indicating that the prediction value is within the allowable range. Thus, according to the data recording apparatus 100 according to the present embodiment, by using the identification information, it is possible to prevent the measurement data serving as a prediction source essential for reproduction from being erroneously deleted.

In addition, the data recording apparatus 100 according to the present embodiment may be capable of setting the allowable range to a range different for each sensor. Thus, according to the data recording apparatus 100 of the present embodiment, whether the acquired measurement data can be predicted (reproduced) can be determined by using different criteria for each sensor. This is particularly effective when the data recording apparatus 100 handles measurement data measured with various precision from various sensors capable of measuring various types of physical quantities.

In addition, the data recording apparatus 100 according to the present embodiment may delete the measurement data acquired from the sensors not included in the set of sensors and record only the measurement data acquired from the sensors included in the set. Thus, according to the data recording apparatus 100 according to the present embodiment, since the measurement data is sorted out depending on whether the measurement data is included in the set, the capacity of the measurement data to be recorded can be greatly reduced.

In addition, the data recording apparatus 100 according to the present embodiment may record the measurement data acquired from the sensor not included in the set of sensors after reducing the number of pieces of data per unit time or reducing the data size per piece of data. Thus, according to the data recording apparatus 100 according to the present embodiment, even when the sensor is not included in the set, it is possible to delete only a part of the measurement data instead of deleting all of the measurement data and continue recording. In addition, according to the data recording apparatus 100 according to the present embodiment, at least a part of the measurement data in any one of a time axis direction or a size axis direction can be deleted selectively according to the characteristic of the measurement data.

In addition, when the remaining capacity available for recording falls below the predetermined threshold value or in response to the elapse of the predetermined time, the data recording apparatus 100 according to the present embodiment may selectively record the measurement data. Thus, the data recording apparatus 100 according to the present embodiment can selectively record the measurement data at timing when it is desired to reduce the total capacity of the measurement data to be recorded.

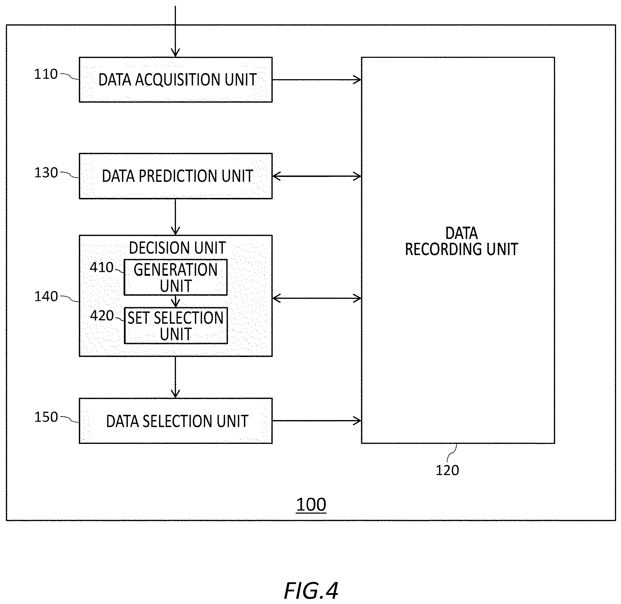

Fig. 4 illustrates an example of a block diagram of the data recording apparatus 100 according to a modification of the present embodiment. In Fig. 4, members having the same functions and configurations as those in Fig. 1 are denoted by the same reference numerals, and description thereof will be omitted except for differences. In the above-described embodiment, a case where the decision unit 140 decides only one set of sensors has been described as an example. However, in the present modification, the decision unit 140 generates a plurality of candidate sets, and selects, as the set of sensors, one candidate set from the plurality of candidate sets. In the data recording apparatus 100 according to the present modification, the decision unit 140 includes a generation unit 410 and a set selection unit 420.

The generation unit 410 generates a plurality of candidate sets of sensors capable of reproducing measurement data acquired from a plurality of sensors within a predetermined allowable range. At this time, the generation unit 410 may generate the plurality of candidate sets by various methods. For example, the generation unit 410 may generate the plurality of candidate sets by randomly reassigning i indicating an index of a sensor to the plurality of sensors and executing the flow illustrated in Fig. 3 a plurality of times. In this case, the generation unit 410 may include the function of the assignment unit 145. However, the present invention is not limited to this. For example, the generation unit 410 may generate the plurality of candidate sets on the basis of a round robin of patterns in which a prediction value, which is obtained by predicting measurement data acquired from at least any one sensor by using measurement data acquired from another sensor, is within the allowable range. This will be described below in detail. The generation unit 410 supplies, to the set selection unit 420, information indicating the generated plurality of candidate sets.

The set selection unit 420 selects a set from the plurality of candidate sets according to a predetermined criterion. At this time, the set selection unit 420 may select a set in consideration of various conditions. For example, the set selection unit 420 may select, among the plurality of candidate sets, a candidate set in which the amount of measurement data to be recorded is small in preference to a candidate set in which the amount is large. Alternatively or additionally, the set selection unit 420 may select, among the plurality of candidate sets, a candidate set in which a difference between the acquired measurement data and the predicted measurement data is small in preference to a candidate set in which the difference is large. This will be described in detail.

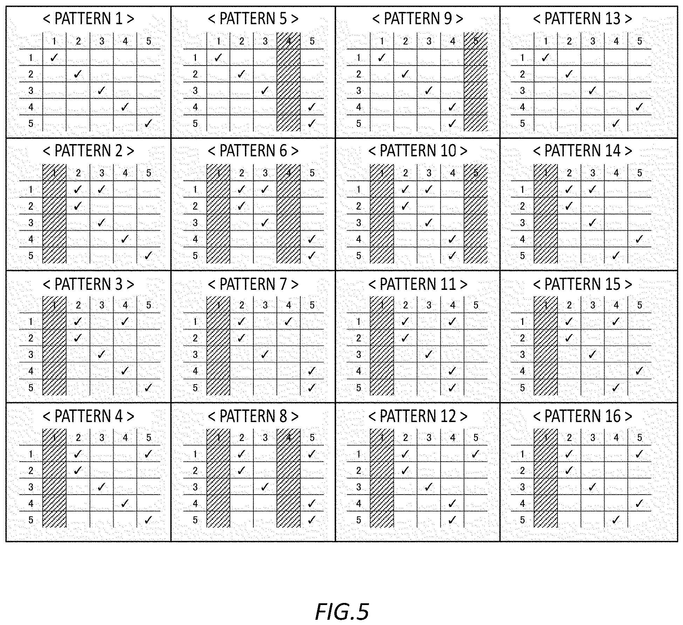

Fig. 5 illustrates an example of the round robin of patterns which may be considered by the data recording apparatus 100 according to the modification of the present embodiment. In each table of the present drawing, a vertical axis and a horizontal axis indicate the index of the sensor. In addition, in each table of the present drawing, a check mark indicates a flag indicating that reproduction is possible within the allowable range. In addition, in each table of the present drawing, a hatched portion indicates data (data having a low priority of recording) which may be deleted since the measurement data acquired from the sensor indicated in the column of the hatched portion can be reproduced within the allowable range.

In the present drawing, a case where the measurement data acquired from the sensor 1 can be reproduced within the allowable range by using any of the measurement data acquired from the sensors 2 and 3, the measurement data acquired from the sensors 2 and 4, or the measurement data acquired from the sensors 2 and 5, the measurement data acquired from the sensor 4 can be reproduced within the allowable range by using the measurement data acquired from the sensor 5, and the measurement data acquired from the sensor 5 can be reproduced within the allowable range by using the measurement data acquired from the sensor 4 is illustrated as an example.

First, focusing on a table of <Pattern 1>, this table shows a case where the measurement data acquired from the sensors 1 to 5 is used as it is (recorded as it is). Of course, since the measurement data acquired from the sensor 1 can be reproduced within the allowable range by using the measurement data acquired from the sensor 1, a cell of (row, column) = (1,1) is flagged. The same applies to the sensor 2 to 4.

Next, focusing on a table of <Pattern 2>, the table shows a case where the measurement data acquired from the sensors 2 to 5 is used as it is while the measurement data acquired from the sensor 1 is reproduced by using the measurement data acquired from the sensors 2 and 3. Since the measurement data acquired from the sensor 1 can be reproduced within the allowable range by using the measurement data acquired from the sensors 2 and 3, cells of (row, column) = (1,2) and (row, column) = (1,3) are flagged. In this case, since the measurement data acquired from the sensor 1 is data that may be deleted, a cell of column = 1 is hatched.

Similarly, focusing on a table of <Pattern 8>, the table shows a case where the measurement data acquired from the sensors 2, 3, and 5 is used as it is, the measurement data acquired from the sensor 1 is reproduced by using the measurement data acquired from the sensors 2 and 5, and the measurement data acquired from the sensor 4 is reproduced using the measurement data acquired from the sensor 5. Since the measurement data acquired from the sensor 1 can be reproduced within the allowable range by using the measurement data acquired from the sensors 2 and 5, cells of (row, column) = (1,2) and (row, column) = (1,5) are flagged. Similarly, since the measurement data acquired from the sensor 4 can be reproduced within the allowable range by using the measurement data acquired from the sensor 5, a cell of (row, column) = (4,5) is flagged. In this case, since the measurement data acquired from the sensor 1 is data that may be deleted, a cell of column = 1 is hatched. Similarly, since the measurement data acquired from the sensor 4 is data that may be deleted, a cell of column = 4 is hatched. The same applies to other patterns.

As described above, there may be a plurality of patterns in which the prediction value obtained by predicting the measurement data acquired from at least any one sensor by using the measurement data acquired from another sensor is within the allowable range. In this case, the generation unit 410 may generate the plurality of candidate sets on the basis of the round robin of the patterns in which the prediction value is within the allowable range. As an example, when the measurement data acquired from the sensor 1 can be reproduced within the allowable range by using any of the measurement data acquired from the sensors 2 and 3, the measurement data acquired from the sensors 2 and 4, or the measurement data acquired from the sensors 2 and 5, the measurement data acquired from the sensor 4 can be reproduced within the allowable range by using the measurement data acquired from the sensor 5, and the measurement data acquired from the sensor 5 can be reproduced within the allowable range by using the measurement data acquired from the sensor 4, a combination of patterns exists in ways of 4×1×1×2×2 = 16.

In this case, the generation unit 410 may generate the plurality of candidate sets on the basis of the 16 ways of patterns. For example, the generation unit 410 may generate a candidate set A including the sensors 2, 3, 4, and 5 on the basis of patterns 2, 3, 4, 7, 11, 12, 14, 15, and 16. In addition, the generation unit 410 may generate a candidate set B including the sensors 1, 2, 3, and 5 on the basis of a pattern 5. In addition, the generation unit 410 may generate a candidate set C including the sensors 2, 3, and 5 on the basis of patterns 6 and 8. In addition, the generation unit 410 may generate a candidate set D including the sensors 1, 2, 3, and 4 on the basis of a pattern 9. In addition, the generation unit 410 may generate a candidate set E including the sensors 2, 3, and 4 on the basis of a pattern 10.

For example, the set selection unit 420 may select, as the set of sensors, one candidate set from among the plurality of candidate sets A to E generated in this manner in consideration of various conditions. At this time, the set selection unit 420 may select, among the plurality of candidate sets, a candidate set in which the amount of measurement data to be recorded is small in preference to a candidate set in which the amount is large. For example, the set selection unit 420 may select a candidate set in which the number of sensors included in the set is small in preference to a candidate set in which the number of sensors is large. As an example, when the candidate set A includes four sensors, the candidate set B includes four sensors, the candidate set C includes three sensors, the candidate set D includes four sensors, and the candidate set E includes three sensors, the set selection unit 420 may select the candidate sets C and E in preference to the candidate sets A, B, and D.

In addition, the set selection unit 420 may select a candidate set in which the total amount of the measurement data acquired from the sensors included in the set is small in preference to a candidate set in which the total amount of the measurement data is large. As an example, it is assumed that the candidate set C includes the sensors 2, 3, and 5, and the candidate set E includes the sensors 2, 3, and 4. In this case, it is assumed that the amount of the measurement data acquired from the sensor 5 is smaller than the amount of the measurement data acquired from the sensor 4. In this case, the set selection unit 420 may select the candidate set C in preference to the candidate set E. At this time, the set selection unit 420 may estimate the amount of measurement data for each sensor on the basis of at least any one of the number of pieces of data per unit time or the data size per piece of data.

In addition, the set selection unit 420 may select, among the plurality of candidate sets, a candidate set in which a difference between the acquired measurement data and the predicted measurement data is small in preference to a candidate set in which the difference is large. As an example, it is assumed that the candidate set C includes the sensors 2, 3, and 5, and the candidate set E includes the sensors 2, 3, and 4. In this case, it is assumed that an error obtained by predicting the measurement data acquired from the sensor 5 by using the measurement data acquired from the sensor 4 is smaller than an error obtained by predicting the measurement data acquired from the sensor 4 by using the measurement data acquired from the sensor 5. In this case, the set selection unit 420 may select the candidate set E in preference to the candidate set C.

As described above, the data recording apparatus 100 according to the present modification may generate a plurality of candidate sets and select, as the set of sensors, one candidate set from the plurality of candidate sets. Thus, according to the data recording apparatus 100 of the present modification, the most preferable candidate set among the plurality of candidate sets can be adopted as the set of sensors.

In addition, the data recording apparatus 100 according to the present modification may generate the plurality of candidate sets on the basis of the round robin of patterns in which the prediction value is within the allowable range. Thus, according to the data recording apparatus 100 of the present modification, an optimal candidate set can be adopted as the set of sensors in consideration of all possible combinations.

In addition, the data recording apparatus 100 according to the present modification may select a candidate set in which the amount of measurement data and the error are small in preference to a candidate set in which the amount and the error are large. Thus, according to the data recording apparatus 100 of the present modification, it is possible to efficiently reduce the amount of measurement data to be recorded and to selectively record measurement data with high reproducibility.

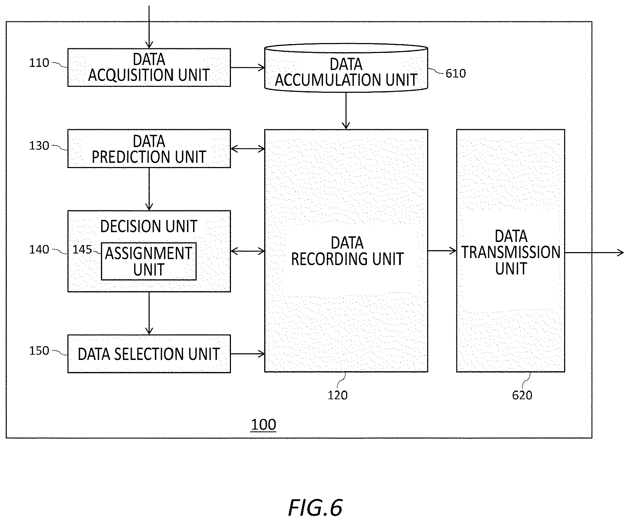

Fig. 6 illustrates an example of a block diagram of the data recording apparatus 100 according to another modification of the present embodiment. In Fig. 6, members having the same functions and configurations as those in Fig. 1 are denoted by the same reference numerals, and description thereof will be omitted except for differences. The data recording apparatus 100 according to the present modification may be provided in an OT region, for example, and may be capable of transmitting measurement data acquired from a sensor provided in the OT region to another system or apparatus provided in an IT region. The data recording apparatus 100 according to the present modification further includes a data accumulation unit 610 and a data transmission unit 620. In addition, in the data recording apparatus 100 according to the present modification, the data acquisition unit 110 supplies the measurement data acquired from the plurality of sensors to the data accumulation unit 610 instead of the data recording unit 120.

The data accumulation unit 610 accumulates measurement data. As an example, the data accumulation unit 610 may accumulate all the measurement data acquired by the data acquisition unit 110 in time series for each sensor. Then, the data accumulation unit 610 supplies, to the data recording unit 120, the measurement data to be transmitted to another system or apparatus among the accumulated measurement data. Note that such a transmission target may be, for example, one selected on the basis of a user input or one automatically selected by the data recording apparatus 100.

Therefore, in the data recording apparatus 100 according to the present modification, the data selection unit 150 selects, among the measurement data to be transmitted, the measurement data acquired from the sensor included in the set decided by the decision unit 140 in preference to the measurement data acquired from the sensor not included in the set. In response to this, the data recording unit 120 records the measurement data selected by the data selection unit 150 among the measurement data to be transmitted.

Then, the data transmission unit 620 transmits, via a network or the like to another system or apparatus, the measurement data recorded in the data recording unit 120 in this manner, that is, the measurement data selected from the measurement data to be transmitted.

As described above, the data recording apparatus 100 according to the present modification may selectively record the measurement data to be transmitted. Thus, according to the data recording apparatus 100 of the present modification, for example, when the measurement data is transmitted from the OT region to the IT region, the amount of data transmitted from the data recording apparatus 100 can be reduced. That is, in a broad sense, selectively recording the measurement data may be interpreted to include excluding unselected measurement data from a target to be transmitted to another system or apparatus in addition to completely deleting the measurement data from the recording region.

Hereinbefore, the embodiments that can be implemented have been described above by way of example. However, the above-described embodiment may be modified or applied in various forms. For example, a sensor capable of predicting (reproducing) measurement data may not only require recording of the measurement data, but also require no maintenance even in the case of failure. Therefore, the data recording apparatus 100 may further include a functional unit that notifies a user or the like of the sensor available for predicting measurement data, in addition to the functional unit included in the data recording apparatus 100 according to the above-described embodiment. In this case, the data recording apparatus 100 may display and output, on the monitor, information for identifying a sensor not included in the decided set, may output the information by sound through a speaker, may output the information by printing through a printer, or may transmit and output the information by sending a message to another apparatus.

Various embodiments of the present invention may be described with reference to flowcharts and block diagrams whose blocks may represent (1) steps of processes in which operations are performed or (2) sections of apparatuses responsible for performing operations. Certain steps and sections may be implemented by dedicated circuitry, programmable circuitry supplied with computer-readable instructions stored on computer-readable media, and/or processors supplied with computer-readable instructions stored on computer-readable media. Dedicated circuitry may include digital and/or analog hardware circuits, and may include integrated circuits (IC) and/or discrete circuits. The programmable circuit may include a reconfigurable hardware circuit including logical AND, logical OR, logical XOR, logical NAND, logical NOR, and other logical operations, a memory element such as a flip-flop, a register, a field programmable gate array (FPGA) and a programmable logic array (PLA), and the like.

A computer-readable medium may include any tangible device that can store instructions to be executed by a suitable device, and as a result, the computer-readable medium having instructions stored thereon includes an article of manufacture including instructions which can be executed to create means for performing operations specified in the flowcharts or block diagrams. Examples of the computer-readable medium may include an electronic storage medium, a magnetic storage medium, an optical storage medium, an electromagnetic storage medium, a semiconductor storage medium, and the like. More specific examples of the computer-readable medium may include a floppy (registered trademark) disk, a diskette, a hard disk, a random access memory (RAM), a read-only memory (ROM), an erasable programmable read-only memory (EPROM or flash memory), an electrically erasable programmable read-only memory (EEPROM), a static random access memory (SRAM), a compact disc read-only memory (CD-ROM), a digital versatile disk (DVD), a Blu-ray (registered trademark) disk, a memory stick, an integrated circuit card, and the like.

The computer-readable instruction may include: an assembler instruction, an instruction-set-architecture (ISA) instruction; a machine instruction; a machine dependent instruction; a microcode; a firmware instruction; state-setting data; or either a source code or an object code written in any combination of one or more programming languages, including an object oriented programming language such as Smalltalk (registered trademark), JAVA (registered trademark), C++, or the like; and a conventional procedural programming language such as a "C" programming language or a similar programming language.

The computer-readable instruction may be provided to a processor of a general purpose computer, special purpose computer, or other programmable data processing apparatuses, or to a programmable circuit, locally or via a local area network (LAN), wide area network (WAN) such as the Internet, or the like, to execute the computer-readable instructions to create means for performing operations specified in the flowcharts or block diagrams. An example of the processor includes a computer processor, processing unit, microprocessor, digital signal processor, controller, microcontroller, etc.

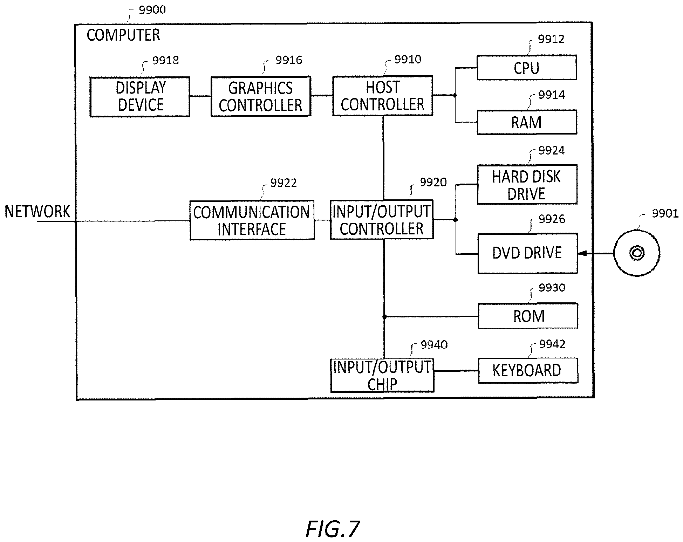

Fig. 7 illustrates an example of a computer 9900 in which a plurality of aspects of the present invention may be embodied in whole or in part. A program that is installed in the computer 9900 can cause the computer 9900 to function as or execute operations associated with the apparatus of the embodiment of the present invention or one or more sections of the apparatus, and/or cause the computer 9900 to execute the processes of the embodiment of the present invention or steps thereof. Such a program may be executed by a CPU 9912 so as to cause the computer 9900 to execute certain operations associated with some or all of the flowcharts and the blocks in the block diagrams described herein.

The computer 9900 according to the present embodiment includes the CPU 9912, a RAM 9914, a graphics controller 9916 and a display device 9918, which are mutually connected by a host controller 9910. The computer 9900 further includes input/output units such as a communication interface 9922, a hard disk drive 9924, a DVD drive 9926 and an IC card drive, which are connected to the host controller 9910 via an input/output controller 9920. The computer also includes legacy input/output units such as a ROM 9930 and a keyboard 9942, which are connected to the input/output controller 9920 via an input/output chip 9940.

The CPU 9912 operates according to programs stored in the ROM 9930 and the RAM 9914, thereby controlling each unit. The graphics controller 9916 acquires image data generated by the CPU 9912 on a frame buffer or the like provided in the RAM 9914 or in itself, and to cause the image data to be displayed on the display device 9918.

The communication interface 9922 communicates with other electronic devices via a network. The hard disk drive 9924 stores programs and data that are used by the CPU 9912 within the computer 9900. The DVD drive 9926 reads programs or data from a DVD-ROM 9901, and to provide the hard disk drive 9924 with the programs or data via the RAM 9914. The IC card drive reads the programs and the data from the IC card, and/or writes the programs and the data to the IC card.

The ROM 9930 stores therein a boot program or the like executed by the computer 9900 at the time of activation, and/or a program depending on the hardware of the computer 9900. The input/output chip 9940 may also connect various input/output units via a parallel port, a serial port, a keyboard port, a mouse port or the like to the input/output controller 9920.

A program is provided by a computer readable storage medium such as the DVD-ROM 9901 or the IC card. The program is read from the computer readable storage medium, installed into the hard disk drive 9924, RAM 9914, ROM 9930, which are also examples of a computer readable storage medium, and executed by CPU 9912. The information processing described in these programs is read into the computer 9900, resulting in cooperation between a program and the above-mentioned various types of hardware resources. An apparatus or method may be constituted by realizing the operation or processing of information in accordance with the usage of the computer 9900.

For example, when communication is performed between the computer 9900 and an external device, the CPU 9912 may execute a communication program loaded onto the RAM 9914 to instruct communication processing to the communication interface 9922, based on the processing described in the communication program. The communication interface 9922, under control of the CPU 9912, reads transmission data stored on a transmission buffer region provided in a recording medium such as the RAM 9914, the hard disk drive 9924, DVD-ROM 9901, or the IC card, and transmits the read transmission data to a network or writes reception data received from a network to a reception buffer region or the like provided on the recording medium.

Also the CPU 9912 may cause all or a necessary portion of a file or a database to be read into the RAM 9914, wherein the file or the database has been stored in an external recording medium such as the hard disk drive 9924, the DVD drive 9926 (DVD-ROM 9901), the IC card, etc., and perform various types of processing on the data on the RAM 9914. The CPU 9912 then writes back the processed data to the external recording medium.

Various types of information such as various types of programs, data, tables, and databases may be stored in a recording medium and subjected to information processing. The CPU 9912 may perform various types of processing on the data read from the RAM 9914, which includes various types of operations, information processing, condition judging, conditional branch, unconditional branch, search/replacement of information, etc., as described throughout this disclosure and designated by an instruction sequence of programs, and writes the result back to the RAM 9914. Also the CPU 9912 may search for information in a file, a database, etc., in the recording medium. For example, when a plurality of entries, each having an attribute value of a first attribute associated with an attribute value of a second attribute, are stored in the recording medium, the CPU 9912 may search for an entry whose attribute value of the first attribute matches the condition a designated condition, from among the plurality of entries, and read the attribute value of the second attribute stored in the entry, thereby obtaining the attribute value of the second attribute associated with the first attribute satisfying the predetermined condition.

The above described program or software modules may be stored in the computer readable storage medium on or near the computer 9900. Also a recording medium such as a hard disk or a RAM provided in a server system connected to a dedicated communication network or the Internet can be used as the computer readable storage medium, thereby providing the program to the computer 9900 via the network.

While the present invention has been described with the embodiments, the technical scope of the present invention is not limited to the scope of the above described embodiments but solely to the appended claims. It is apparent to persons skilled in the art that varied alteration or improvement can be added to the above embodiments. It is apparent from description of the claims that a form added with such alteration or improvement may also be included in the technical scope of the present invention as long as it falls within the appended claims.

It should be noted that each processing such as the operations, procedures, steps, and stages in the apparatus, system, program, and method shown in the claims, the specification, or the drawings may be realized in any order, unless its execution order is specified as "before", "prior to", or the like and unless output from previous processing is used in subsequent processing. Even if description is made, regarding an operation flow in the claims, the specification or the drawings, by using "first", "next", or the like as a matter of convenience, it does not necessarily mean that the processing must be performed in this order.

### EXPLANATION OF REFERENCES

100: data recording apparatus;
110: data acquisition unit;
120: data recording unit;
130: data prediction unit;
140: decision unit;
145: assignment unit;
150: data selection unit;
410: generation unit;
420: set selection unit;
610: data accumulation unit;
620: data transmission unit;
9900: computer;
9901: DVD-ROM;
9910: host controller;
9912: CPU;
9914: RAM;
9916: graphic controller;
9918: display device;
9920: input/output controller;
9922: communication interface;
9924: hard disk drive;
9926: DVD drive;
9930: ROM;
9940: input/output chip; and
9942: keyboard.

## Claims

1. A data recording apparatus (100) comprising:
a data acquisition unit (110) configured to acquire measurement data obtained by measuring a measurement target from a plurality of sensors (1, 2, 3,4,5);
a data prediction unit (130) configured to predict measurement data acquired from at least any one sensor (1, 2, 3,4,5) of the plurality of sensors (1, 2, 3,4,5) by using measurement data acquired from another sensor (1, 2, 3,4,5) among the plurality of sensors (1, 2, 3,4,5);
a decision unit (140) configured to decide a set of sensors (1, 2, 3,4,5) capable of reproducing the measurement data acquired from the plurality of sensors (1, 2, 3,4,5) within a predetermined allowable range by using the measurement data acquired by the data acquisition unit (110) and the measurement data predicted by the data prediction unit (130), wherein measurement data acquired from a sensor (1, 2, 3,4,5) not included in the set can be reproduced within the predetermined allowable range by using the measurement data acquired from one or more sensors (1, 2, 3,4,5) in the set;
a data selection unit (150) configured to select, from among the measurement data acquired from the plurality of sensors (1, 2, 3,4,5), the measurement data acquired from a sensor (1, 2, 3,4,5) included in the set in preference to the measurement data acquired from a sensor (1, 2, 3,4,5) not included in the set; and
a data recording unit (120) configured to record the measurement data selected by the data selection unit, wherein the measurement data selected by the data selection unit (150) include the measurement data acquired from sensors (1, 2, 3,4,5) included in the set.

2. The data recording apparatus (100) according to claim 1, wherein
the decision unit (140) has an assignment unit (145) configured to assign, to the another sensor (1, 2, 3,4,5), identification information indicating whether a prediction value, which is obtained by predicting the measurement data acquired from the at least any one sensor (1, 2, 3,4,5) by using the measurement data acquired from the another sensor (1, 2, 3,4,5), is within the allowable range, and
a sensor (1, 2, 3,4,5) which is assigned with the identification information indicating that the prediction value is within the allowable range is included in the set.

3. The data recording apparatus (100) according to claim 1, wherein
the decision unit (140) has:
a generation unit (410) configured to generate a plurality of candidate sets of sensors (1, 2, 3,4,5) capable of reproducing the measurement data acquired from the plurality of sensors (1, 2, 3,4,5) within a predetermined allowable range; and
a set selection unit (420) configured to select the set from the plurality of candidate sets according to a predetermined criterion.

4. The data recording apparatus (100) according to claim 3, wherein the generation unit (410) is configured to generate the plurality of candidate sets on a basis of a round robin of patterns in which a prediction value obtained by predicting the measurement data acquired from the at least any one sensor (1, 2, 3,4,5) using the measurement data acquired from the another sensor (1, 2, 3,4,5) is within the allowable range.

5. The data recording apparatus (100) according to claim 3 or 4, wherein the set selection unit (420) is configured to select, among the plurality of candidate sets, a candidate set in which an amount of the measurement data to be recorded is small in preference to a candidate set in which the amount is large.

6. The data recording apparatus (100) according to claim 3 or 4, wherein the set selection unit (420) is configured to select, among the plurality of candidate sets, a candidate set in which a difference between the measurement data acquired by the data acquisition unit (110) and the measurement data predicted by the data prediction unit (130) is small in preference to a candidate set in which the difference is large.

7. The data recording apparatus (100) according to any one of claims 1 to 4, wherein the allowable range is settable to a range different for each sensor (1, 2, 3,4,5).

8. The data recording apparatus (100) according to any one of claims 1 to 4, wherein the data recording unit (120) is configured to delete the measurement data acquired from the sensor (1, 2, 3,4,5) not included in the set and record only the measurement data acquired from the sensor (1, 2, 3,4,5) included in the set.

9. The data recording apparatus (100) according to any one of claims 1 to 4, wherein the data recording unit (120) is configured to record the measurement data acquired from the sensor (1, 2, 3,4,5) not included in the set after reducing a number of at least one piece of data per unit time.

10. The data recording apparatus (100) according to any one of claims 1 to 4, wherein the data recording unit (120) is configured to record the measurement data acquired from the sensor (1, 2, 3,4,5) not included in the set after reducing a data size per piece of data.

11. The data recording apparatus (100) according to any one of claims 1 to 4, wherein the data recording unit (120) is configured to record the measurement data selected by the data selection unit (150) in response to a remaining capacity available for recording falling below a predetermined threshold value.

12. The data recording apparatus (100) according to any one of claims 1 to 4, wherein the data recording unit (120) is configured to record the measurement data selected by the data selection unit (150) in response to elapse of predetermined time.

13. The data recording apparatus (100) according to any one of claims 1 to 4, further comprising a data transmission unit (620) configured to transmit the measurement data selected by the data selection unit (150) to another system or apparatus.

14. A data recording method executed by a computer (9900), comprising: by the computer (9900)
acquiring measurement data obtained by measuring a measurement target from a plurality of sensors (1, 2, 3,4,5);
predicting measurement data acquired from at least any one sensor (1, 2, 3,4,5) of the plurality of sensors (1, 2, 3,4,5) by using measurement data acquired from another sensor (1, 2, 3,4,5) among the plurality of sensors (1, 2, 3,4,5);
deciding a set of sensors (1, 2, 3,4,5) capable of reproducing the measurement data acquired from the plurality of sensors (1, 2, 3,4,5) within a predetermined allowable range by using the measurement data acquired by the acquiring and the measurement data predicted by the predicting, wherein measurement data acquired from a sensor (1, 2, 3,4,5) not included in the set can be reproduced within the predetermined allowable range by using the measurement data acquired from one or more sensors (1, 2, 3,4,5) in the set;
selecting, from among the measurement data acquired from the plurality of sensors (1, 2, 3,4,5), the measurement data acquired from a sensor (1, 2, 3,4,5) included in the set in preference to the measurement data acquired from a sensor (1, 2, 3,4,5) not included in the set; and
recording the measurement data selected by the selecting, wherein the measurement data selected by the selecting include the measurement data acquired from sensors (1, 2, 3,4,5) included in the set.

15. A data recording program that, when executed by a computer (9900), causes the computer (9900) to carry out the method of claim 14.

## Patentansprüche

1. Eine Datenaufzeichnungsvorrichtung (100), die folgende Merkmale aufweist:
eine Datenerfassungseinheit (110), die dazu konfiguriert ist, Messdaten zu erfassen, die durch Messen eines Messziels von einer Mehrzahl von Sensoren (1, 2, 3, 4, 5) erhalten werden;
eine Datenvorhersageeinheit (130), die dazu konfiguriert ist, Messdaten vorherzusagen, die von zumindest einem Sensor (1, 2, 3, 4, 5) der Mehrzahl von Sensoren (1, 2, 3, 4, 5) erfasst werden, unter Verwendung von Messdaten, die von einem anderen Sensor (1, 2, 3, 4, 5) unter der Mehrzahl von Sensoren (1, 2, 3, 4, 5) erfasst werden;
eine Entscheidungseinheit (140), die dazu konfiguriert ist, einen Satz von Sensoren (1, 2, 3, 4, 5) zu bestimmen, die in der Lage sind, die Messdaten, die von der Mehrzahl von Sensoren (1, 2, 3, 4, 5) erfasst werden, innerhalb eines vorbestimmten zulässigen Bereichs wiederzugeben unter Verwendung der Messdaten, die von der Datenerfassungseinheit (110) erfasst werden, und der Messdaten, die von der Datenvorhersageeinheit (130) vorhergesagt werden, wobei Messdaten, die von einem Sensor (1, 2, 3, 4, 5) erfasst werden, der nicht in dem Satz enthalten ist, innerhalb des vorbestimmten zulässigen Bereichs wiedergegeben werden können unter Verwendung der Messdaten, die von einem oder mehreren Sensoren (1, 2, 3, 4, 5) in dem Satz erfasst werden;
eine Datenauswahleinheit (150), die dazu konfiguriert ist, unter den Messdaten, die von der Mehrzahl von Sensoren (1, 2, 3, 4, 5) erfasst werden, die Messdaten auszuwählen, die von einem Sensor (1, 2, 3, 4, 5) erfasst werden, der in dem Satz enthalten ist, anstelle von den Messdaten, die von einem Sensor (1, 2, 3, 4, 5) erfasst werden, der nicht in dem Satz enthalten ist; und
eine Datenaufzeichnungseinheit (120), die dazu konfiguriert ist, die Messdaten, die von der Datenauswahleinheit ausgewählt werden, aufzuzeichnen, wobei die Messdaten, die von der Datenauswahleinheit (150) ausgewählt werden, die Messdaten enthalten, die von Sensoren (1, 2, 3, 4, 5) erfasst werden, die in dem Satz enthalten sind.

2. Die Datenaufzeichnungsvorrichtung (100) gemäß Anspruch 1, bei der
die Entscheidungseinheit (140) eine Zuweisungseinheit (145) aufweist, die dazu konfiguriert ist, dem anderen Sensor (1, 2, 3, 4, 5) Identifikationsinformationen zuzuweisen, die angeben, ob ein Vorhersagewert, der durch Vorhersagen der Messdaten erhalten wird, die von dem zumindest einen Sensor (1, 2, 3, 4, 5) erfasst werden, innerhalb des zulässigen Bereichs liegt, unter Verwendung der Messdaten, die von dem anderen Sensor (1, 2, 3, 4, 5) erfasst werden, und
ein Sensor (1, 2, 3, 4, 5), dem die Identifikationsinformationen zugewiesen sind, die angeben, dass der Vorhersagewert innerhalb des zulässigen Bereichs liegt, in dem Satz enthalten ist.

3. Die Datenaufzeichnungsvorrichtung (100) gemäß Anspruch 1, bei der
die Entscheidungseinheit (140) folgende Merkmale aufweist:
eine Erzeugungseinheit (410), die dazu konfiguriert ist, eine Mehrzahl von Kandidatensätzen von Sensoren (1, 2, 3, 4, 5) zu erzeugen, die in der Lage sind, die Messdaten, die von der Mehrzahl von Sensoren (1, 2, 3, 4, 5) erfasst werden, innerhalb eines vorbestimmten zulässigen Bereichs wiederzugeben; und
eine Satzauswahleinheit (420), die dazu konfiguriert ist, den Satz aus der Mehrzahl von Kandidatensätzen gemäß einem vorbestimmten Kriterium auszuwählen.

4. Die Datenaufzeichnungsvorrichtung (100) gemäß Anspruch 3, bei der die Erzeugungseinheit (410) dazu konfiguriert ist, die Mehrzahl von Kandidatensätzen auf einer Basis eines Round-Robin von Mustern zu erzeugen, bei dem ein Vorhersagewert, der durch Vorhersagen der Messdaten erhalten wird, die von dem zumindest einen Sensor (1, 2, 3, 4, 5) erfasst werden, innerhalb des zulässigen Bereichs liegt, unter Verwendung der Messdaten, die von dem anderen Sensor (1, 2, 3, 4, 5) erfasst werden.

5. Die Datenaufzeichnungsvorrichtung (100) gemäß Anspruch 3 oder 4, bei der die Satzauswahleinheit (420) dazu konfiguriert ist, unter der Mehrzahl von Kandidatensätzen einen Kandidatensatz auszuwählen, bei dem eine Menge der Messdaten, die aufzuzeichnen ist, klein ist, anstelle von einem Kandidatensatz, bei dem die Menge groß ist.

6. Die Datenaufzeichnungsvorrichtung (100) gemäß Anspruch 3 oder 4, bei der die Satzauswahleinheit (420) dazu konfiguriert ist, unter der Mehrzahl von Kandidatensätzen einen Kandidatensatz auszuwählen, bei dem eine Differenz zwischen den Messdaten, die von der Datenerfassungseinheit (110) erfasst werden, und den Messdaten, die von der Datenvorhersageeinheit (130) vorhergesagt werden, klein ist, anstelle von einem Kandidatensatz, bei dem die Differenz groß ist.

7. Die Datenaufzeichnungsvorrichtung (100) gemäß einem der Ansprüche 1 bis 4, bei der der zulässige Bereich auf einen Bereich einstellbar ist, der für jeden Sensor (1, 2, 3, 4, 5) unterschiedlich ist.

8. Die Datenaufzeichnungsvorrichtung (100) gemäß einem der Ansprüche 1 bis 4, bei der die Datenaufzeichnungseinheit (120) dazu konfiguriert ist, die Messdaten, die von dem Sensor (1, 2, 3, 4, 5) erfasst werden, der nicht in dem Satz enthalten ist, zu löschen und nur die Messdaten, die von dem Sensor (1, 2, 3, 4, 5) erfasst werden, der in dem Satz enthalten ist, aufzuzeichnen.

9. Die Datenaufzeichnungsvorrichtung (100) gemäß einem der Ansprüche 1 bis 4, bei der die Datenaufzeichnungseinheit (120) dazu konfiguriert ist, die Messdaten, die von dem Sensor (1, 2, 3, 4, 5) erfasst werden, der nicht in dem Satz enthalten ist, nach dem Reduzieren einer Anzahl von zumindest einem Datenelement pro Zeiteinheit aufzuzeichnen.

10. Die Datenaufzeichnungsvorrichtung (100) gemäß einem der Ansprüche 1 bis 4, bei der die Datenaufzeichnungseinheit (120) dazu konfiguriert ist, die Messdaten, die von dem Sensor (1, 2, 3, 4, 5) erfasst werden, der nicht in dem Satz enthalten ist, nach dem Reduzieren einer Datengröße pro Datenelement aufzuzeichnen.

11. Die Datenaufzeichnungsvorrichtung (100) gemäß einem der Ansprüche 1 bis 4, bei der die Datenaufzeichnungseinheit (120) dazu konfiguriert ist, die Messdaten, die von der Datenauswahleinheit (150) ausgewählt werden, ansprechend darauf aufzuzeichnen, dass eine verbleibende Kapazität, die zum Aufzeichnen verfügbar ist, unter einen vorbestimmten Schwellenwert fällt.

12. Die Datenaufzeichnungsvorrichtung (100) gemäß einem der Ansprüche 1 bis 4, bei der die Datenaufzeichnungseinheit (120) dazu konfiguriert ist, die Messdaten, die von der Datenauswahleinheit (150) ausgewählt werden, ansprechend auf das Verstreichen einer vorbestimmten Zeit aufzuzeichnen.

13. Die Datenaufzeichnungsvorrichtung (100) gemäß einem der Ansprüche 1 bis 4, die ferner eine Datenübertragungseinheit (620) aufweist, die dazu konfiguriert ist, die Messdaten, die von der Datenauswahleinheit (150) ausgewählt werden, an ein anderes System oder eine andere Vorrichtung zu übertragen.

14. Ein Datenaufzeichnungsverfahren, das von einem Computer (9900) ausgeführt wird, das folgende Schritte aufweist: durch den Computer (9900)
Erfassen von Messdaten, die durch Messen eines Messziels von einer Mehrzahl von Sensoren (1, 2, 3, 4, 5) erhalten werden;
Vorhersagen von Messdaten, die von zumindest einem Sensor (1, 2, 3, 4, 5) der Mehrzahl von Sensoren (1, 2, 3, 4, 5) erfasst werden, unter Verwendung von Messdaten, die von einem anderen Sensor (1, 2, 3, 4, 5) unter der Mehrzahl von Sensoren (1, 2, 3, 4, 5) erfasst werden;
Bestimmen eines Satzes von Sensoren (1, 2, 3, 4, 5), die in der Lage sind, die Messdaten, die von der Mehrzahl von Sensoren (1, 2, 3, 4, 5) erfasst werden, innerhalb eines vorbestimmten zulässigen Bereichs wiederzugeben unter Verwendung der Messdaten, die durch das Erfassen erfasst werden, und der Messdaten, die durch das Vorhersagen vorhergesagt werden, wobei Messdaten, die von einem Sensor (1, 2, 3, 4, 5) erfasst werden, der nicht in dem Satz enthalten ist, innerhalb des vorbestimmten zulässigen Bereichs wiedergegeben werden können unter Verwendung der Messdaten, die von einem oder mehreren Sensoren (1, 2, 3, 4, 5) in dem Satz erfasst werden;
Auswählen, unter den Messdaten, die von der Mehrzahl von Sensoren (1, 2, 3, 4, 5) erfasst werden, der Messdaten, die von einem Sensor (1, 2, 3, 4, 5) erfasst werden, der in dem Satz enthalten ist, anstelle von den Messdaten, die von einem Sensor (1, 2, 3, 4, 5) erfasst werden, der nicht in dem Satz enthalten ist; und
Aufzeichnen der Messdaten, die durch das Auswählen ausgewählt werden, wobei die Messdaten, die durch das Auswählen ausgewählt werden, die Messdaten enthalten, die von Sensoren (1, 2, 3, 4, 5) erfasst werden, die in dem Satz enthalten sind.

15. Ein Datenaufzeichnungsprogramm, das, wenn dasselbe von einem Computer (9900) ausgeführt wird, den Computer (9900) veranlasst, das Verfahren gemäß Anspruch 14 auszuführen.

## Revendications

1. Appareil d'enregistrement de données (100) comprenant:
une unité d'acquisition de données (110) configurée pour acquérir les données de mesure obtenues en mesurant une cible de mesure par une pluralité de capteurs (1, 2, 3, 4, 5);
une unité de prédiction de données (130) configurée pour prédire les données de mesure acquises à partir d'au moins un capteur (1, 2, 3, 4, 5) parmi la pluralité de capteurs (1, 2, 3, 4, 5) à l'aide des données de mesure acquises à partir d'un autre capteur (1, 2, 3, 4, 5) parmi la pluralité de capteurs (1, 2, 3, 4, 5);
une unité de décision (140) configurée pour décider un ensemble de capteurs (1, 2, 3, 4, 5) à même de reproduire les données de mesure acquises à partir de la pluralité de capteurs (1, 2, 3, 4, 5) dans une plage admissible prédéterminée à l'aide des données de mesure acquises par l'unité d'acquisition de données (110) et des données de mesure prédites par l'unité de prédiction de données (130), où les données de mesure acquises à partir d'un capteur (1, 2, 3, 4, 5) non incluses dans l'ensemble peuvent être reproduites dans la plage admissible prédéterminée à l'aide des données de mesure acquises à partir d'un ou plusieurs capteurs (1, 2, 3, 4, 5) dans l'ensemble;
une unité de sélection de données (150) configurée pour sélectionner, parmi les données de mesure acquises à partir de la pluralité de capteurs (1, 2, 3, 4, 5), les données de mesure acquises à partir d'un capteur (1, 2, 3, 4, 5) inclus dans l'ensemble par préférence aux données de mesure acquises à partir d'un capteur (1, 2, 3, 4, 5) non inclus dans l'ensemble; et
une unité d'enregistrement de données (120) configurée pour enregistrer les données de mesure sélectionnées par l'unité de sélection de données, où les données de mesure sélectionnées par l'unité de sélection de données (150) comportent les données de mesure acquises à partir de capteurs (1, 2, 3, 4, 5) inclus dans l'ensemble.

2. Appareil d'enregistrement de données (100) selon la revendication 1, dans lequel
l'unité de décision (140) présente une unité d'attribution (145) configurée pour attribuer à l'autre capteur (1, 2, 3, 4, 5) les informations d'identification indiquant si une valeur de prédiction qui est obtenue en prédisant les données de mesure acquises à partir de l'au moins un capteur (1, 1, 3, 4, 5) à l'aide des données de mesure acquises à partir de l'autre capteur quelconque (1, 2, 3, 4, 5) se situe dans la plage admissible, et
un capteur (1, 2, 3, 4, 5) auquel sont attribuées les informations d'identification indiquant que la valeur de prédiction se situe dans la plage admissible est inclus dans l'ensemble.

3. Appareil d'enregistrement de données (100) selon la revendication 1, dans lequel
l'unité de décision (140) présente:
une unité de génération (410) configurée pour générer une pluralité d'ensembles candidats de capteurs (1, 2, 3, 4, 5) à même de reproduire les données de mesure acquises à partir de la pluralité de capteurs (1, 2, 3, 4, 5) dans une plage admissible prédéterminée; et
une unité de sélection d'ensemble (420) configurée pour sélectionner l'ensemble parmi la pluralité d'ensembles candidats selon un critère prédéterminé.

4. Appareil d'enregistrement de données (100) selon la revendication 3, dans lequel l'unité de génération (410) est configurée pour générer la pluralité d'ensembles candidats sur base d'un tournoi à la ronde de modèles dans lequel une valeur de prédiction obtenue en prédisant les données de mesure acquises à partir de l'au moins un capteur quelconque (1, 2, 3, 4, 5) à l'aide des données de mesure acquises à partir de l'autre capteur (1, 2, 3, 4, 5) se situe dans la plage admissible.

5. Appareil d'enregistrement de données (100) selon la revendication 3 ou 4, dans lequel l'unité de sélection d'ensemble (420) est configurée pour sélectionner, parmi la pluralité d'ensembles candidats, un ensemble candidat dans lequel une quantité de données de mesure à enregistrer est faible par préférence à un ensemble candidat dans lequel la quantité est grande.

6. Appareil d'enregistrement de données (100) selon la revendication 3 ou 4, dans lequel l'unité de sélection d'ensemble (420) est configurée pour sélectionner, parmi la pluralité d'ensembles candidats, un ensemble candidat dans lequel une différence entre les données de mesure acquises par l'unité d'acquisition de données (110) et les données de mesure prédites par l'unité de prédiction de données (130) est faible par préférence à un ensemble candidat dans lequel la différence est grande.

7. Appareil d'enregistrement de données (100) selon l'une quelconque des revendications 1 à 4, dans lequel la plage admissible peut être réglée à une plage différente pour chaque capteur (1, 2, 3, 4, 5).

8. Appareil d'enregistrement de données (100) selon l'une quelconque des revendications 1 à 4, dans lequel l'unité d'enregistrement de données (120) est configurée pour supprimer les données de mesure acquises du capteur (1, 2, 3, 4, 5) non inclus dans l'ensemble et pour enregistrer uniquement les données de mesure acquises du capteur (1, 2, 3, 4, 5) inclus dans l'ensemble.

9. Appareil d'enregistrement de données (100) selon l'une quelconque des revendications 1 à 4, dans lequel l'unité d'enregistrement de données (120) est configurée pour enregistrer les données de mesure acquises du capteur (1, 2, 3, 4, 5) non inclus dans l'ensemble après la réduction d'un nombre d'au moins un élément de données par temps unitaire.

10. Appareil d'enregistrement de données (100) selon l'une quelconque des revendications 1 à 4, dans lequel l'unité d'enregistrement de données (120) est configurée pour enregistrer les données de mesure acquises du capteur (1, 2, 3, 4, 5) non inclus dans l'ensemble après la réduction d'une grandeur de données par élément de données.

11. Appareil d'enregistrement de données (100) selon l'une quelconque des revendications 1 à 4, dans lequel l'unité d'enregistrement de données (120) est configurée pour enregistrer les données de mesure sélectionnées par l'unité de sélection de données (150) en réponse à une capacité restante disponible pour l'enregistrement tombant au-dessous d'une valeur de seuil prédéterminée.

12. Appareil d'enregistrement de données (100) selon l'une quelconque des revendications 1 à 4, dans lequel l'unité d'enregistrement de données (120) est configurée pour enregistrer les données de mesure sélectionnées par l'unité de sélection de données (150) en réponse à l'écoulement d'un temps prédéterminé.

13. Appareil d'enregistrement de données (100) selon l'une quelconque des revendications 1 à 4, comprenant par ailleurs une unité de transmission de données (620) configurée pour transmettre les données de mesure sélectionnées par l'unité de sélection de données (150) à un autre système ou appareil.

14. Procédé d'enregistrement de données réalisé par un ordinateur (9900), comprenant le fait de:
par l'ordinateur (9900),
acquérir les données de mesure obtenues en mesurant une cible de mesure d'une pluralité de capteurs (1, 2, 3, 4, 5);
prédire les données de mesure acquises d'au moins un capteur quelconque (1, 2, 3, 4, 5) parmi la pluralité de capteurs (1, 2, 3, 4, 5) à l'aide des données de mesure acquises d'un autre capteur (1, 2, 3, 4, 5) parmi la pluralité de capteurs (1, 2, 3, 4, 5);
décider un ensemble de capteurs (1, 2, 3, 4, 5) à même de reproduire les données de mesure acquises de la pluralité de capteurs (1, 2, 3, 4, 5) dans une plage admissible prédéterminée à l'aide des données de mesure acquises par l'acquisition et des données prédites par la prédiction, où les données de mesure acquises d'un capteur (1, 2, 3, 4, 5) non inclus dans l'ensemble peuvent être reproduites dans la plage admissible prédéterminée à l'aide des données de mesure acquises d'un ou plusieurs capteurs (1, 2, 3, 4, 5) dans l'ensemble;
sélectionner, parmi les données de mesure acquises de la pluralité de capteurs (1, 2, 3, 4, 5), les données de mesure acquises d'un capteur (1, 2, 3, 4, 5) inclus dans l'ensemble par préférence aux données de mesure acquises d'un capteur (1, 2, 3, 4, 5) non inclus dans l'ensemble; et
enregistrer les données de mesure sélectionnées par la sélection, où les données de mesure sélectionnées par la sélection comportent les données de mesure acquises de capteurs (1, 2, 3, 4, 5) inclus dans l'ensemble.

15. Programme d'enregistrement de données qui, lorsqu'il est exécuté par un ordinateur (9900), amène l'ordinateur (9900) à réaliser le procédé selon la revendication 14.
